# FASCICULE DE BREVET EUROPEEN

(11) **EP 0 756 866 B1**
(45) Date de publication et mention de la délivrance du brevet: **10.12.1997**
(21) Numéro de dépôt: 96401542.4
(22) Date de dépôt: 11.07.1996
(51) Int. Cl.: A61K 7/48

(54) **Utilisation de dérivés d'acide benzoique pour stimuler le processus de renouvellement épidermique et traiter la peau**
Verwendung der Benzosäurederivate zur Stimulierung die Erneuerung der Epidermis und zur Behandlung der Haut
Use of benzoic acid derivatives for stimulating the renewal of the epidermis and for treating the skin

(30) Priorité: 31.07.1995 FR 9509305
(43) Date de publication de la demande: 05.02.1997
(73) Titulaire: L'OREAL, 75008 Paris (FR)
(72) Inventeur: Arraudeau, Jean-Pierre, 75015 Paris (FR); Aubert, Lucien Les Rocailles, 06320 Cap d'Ail (FR)
(74) Mandataire: Andral, Christophe André Louis

(56) Documents cités:
- WO-A-95/03028
- US-A- 3 161 566
- CHEMICAL ABSTRACTS, vol. 124, no. 14, 1 Avril 1996 Columbus, Ohio, US; abstract no. 185112, SHIGA CENTRAL LABORATORIES, NOEVIR CO., LTD.: "Scavenging activities or polyhydroxybenzoic acids on hydroxyl radicals" XP002018491 & J. SCCJ:, vol. 29, no. 3, 1995, pages 270-273, N. OKAMOTO:
- CHEMICAL ABSTRACTS, vol. 120, no. 3, 17 Janvier 1994 Columbus, Ohio, US; abstract no. 24937, Y. KOIKAWA: "antiaging cosmetics containing plant extracts" XP002003247 & JP-A-06 024 937 (NARISU COSMETIC CO LTD.) 1 Février 1994
- PLANTA MEDICA, vol. 57, 1991, page a54 XP000570546 C MONTESINOS: "superoxide scavenging properties of phenolic acids"
- DATABASE WPI Week 9529 Derwent Publications Ltd., London, GB; AN 95-220743 XP002003248 "Skin ageing inhibitor for external use-comprises sugar of 3,4,5-dihydroxybenzoic acid" & JP-A-07 133 218 (NOEVIR KK) , 23 Mai 1995

## Description

L'invention se rapporte à l'utilisation d'acide dihydroxybenzoïque dans ou pour la fabrication d'une composition cosmétique ou dermatologique par application topique sur la peau du visage et/ou du corps, pour stimuler le processus de renouvellement épidermique et/ou lutter contre les rides et/ou ridules et/ou taches de la peau. Elle se rapporte aussi à un procédé de traitement non thérapeutique de la peau destiné au traitement des rides et/ou ridules et/ou taches de la peau.

Il est connu d'utiliser dans les compositions cosmétiques de l'acide dihydroxy-2,5-benzoïque ou acide gentisique, notamment comme agent de blanchiment de la peau (voir le document JP-A-05-43446), comme agent pour combattre les radicaux libres (voir le document C. Montesinos et al., Planta Med., 57, Supplement issue 2, 1991 page A54) et comme anti-oxydant (voir le document JP-A-01-249885 et JP-06-24937). Il est connu également d'utiliser un acide dihydroxybenzoïque pour éliminer les couches durcies ou épaissies de la kératine, constituant les cors et callosités (US-A-3161566).

Or, la demanderesse a trouvé de manière inattendue que l'application topique d'un acide dihydroxybenzoïque, et notamment de l'acide gentisique permettait de stimuler le processus de renouvellement cellulaire épidermique et le processus de réparation épidermique.

Il est connu que les substances qui stimulent le processus de renouvellement cellulaire épidermique ont des effets bénéfiques équivalents à ceux obtenus par un 〈〈 peeling 〉〉 épidermique et sont utilisées pour lutter contre les principaux signes cliniques du vieillissement cutané, notamment contre la formation de rides ou de ridules et contre l'apparition de taches actiniques. La stimulation du renouvellement cellulaire épidermique de la peau est associée à une amélioration clinique de la qualité de la peau qui devient plus éclatante, moins ridée et d'une façon générale plus jeune. Elle permet également une amélioration de l'état acnéique de la peau. On peut citer comme exemples de telles substances les alpha-hydroxy-acides, les bêta-hydroxy-acides tels que l'acide salicylique, les rétinoïdes topiques, etc...

On cherche à diversifier le type de produit de stimulation du renouvellement cellulaire épidermique utilisé et il subsiste donc le besoin d'autres substances ayant des propriétés de stimulation du renouvellement cellulaire épidermique.

Aussi, la présente invention a pour objet l'utilisation d'au moins un acide dihydroxybenzoïque dans ou pour la fabrication d'une composition cosmétique ou dermatologique pour stimuler le processus de renouvellement épidermique.

L'utilisation d'acide dihydroxybenzoïque permet de traiter de façon générale toute imperfection de la peau, notamment les taches, les dyschromies cutanées, les dermites, les lentigos actiniques, les cicatrices et pigmentations cicatricielles, les ichtyoses. Par rapport aux produits de stimulation du renouvellement épidermique connus antérieurement, les acides dihydroxybenzoïques ont l'avantage de présenter en plus d'autres propriétés intéressantes, notamment des propriétés anti-inflammatoires, ce qui en fait des composés moins irritants et donc mieux tolérés.

Aussi, l'invention a encore pour objet l'utilisation d'au moins un acide dihydroxybenzoïque dans ou pour la fabrication d'une composition cosmétique ou dermatologique pour lutter contre les rides et/ou les ridules et/ou les taches actiniques et/ou les dyschromies cutanées et/ou l'ichtyose et/ou l'acné.

Les acides dihydroxybenzoïques selon l'invention sont les composés ayant la formule suivante : 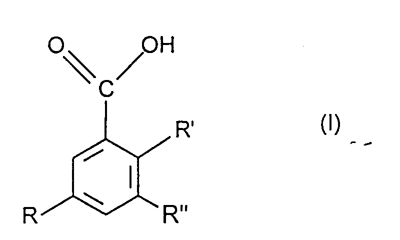 dans laquelle R, R' et R'' représentent indépendamment l'un de l'autre un hydrogène, un hydroxyle, un groupe alkyle ou un groupe alcoxy, deux au moins des radicaux R, R' et R'' représentant un hydroxyle. Les groupes alkyle et alcoxy comportent de préférence de 1 à 23 atomes de carbone et peuvent être en outre saturés ou insaturés.

On peut citer notamment comme acide dihydroxybenzoïque de formule (I) l'acide gentisique et l'acide dihydroxy-2,3-benzoïque. Ces acides peuvent être utilisés tels quels ou sous forme d'extraits naturels en contenant. Comme extrait naturel, on peut citer par exemple la gentiane.

Dans les compositions selon l'invention, l'acide dihydroxybenzoïque (ou un mélange d'acides dihydroxybenzoïques) est utilisé en une quantité allant de 0,1 à 30 % en poids par rapport au poids total de la composition et en particulier en une quantité' allant de 0,2 à 10 % en poids par rapport au poids total de la composition.

En outre, la demanderesse a observé que ces composés peuvent être associés à d'autres actifs connus pour leur propriété de stimulation du renouvellement épidermique, par exemple les hydroxy-acides, les α- ou β-céto-acides ou les rétinoïdes. Une telle association permet de diminuer la concentration active de ces produits du fait de l'addition de leurs effets. On peut ainsi obtenir une composition moins irritante et plus efficace.

Aussi, l'invention a aussi pour objet une composition cosmétique et/ou dermatologique, caractérisée en ce qu'elle contient, dans un milieu physiologiquement acceptable, au moins un acide dihydroxybenzoïque et au moins un actif choisi parmi les α-hydroxy-acides, les β-hydroxy-acides, les α-céto-acides, les β-céto-acides, les rétinoïdes.

Les hydroxyacides peuvent être par exemple des α-hydroxyacides ou des β-hydroxyacides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Les hydroxyacides qui peuvent être utilisés sont notamment les acides glycolique, lactique, malique, tartrique, citrique et de manière générale les acides de fruits, les acides hydroxy-2 alcanoïque, mandélique, salicylique, ainsi que leurs dérivés alkylés comme l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthyl-benzoïque ou encore leurs dérivés alcoxylés comme l'acide 2-hydroxy-3-méthoxybenzoïque.

Les rétinoïdes peuvent être notamment l'acide rétinoïque (all-trans ou 13-cis) et ses dérivés, le rétinol (vitamine A) et ses esters tels que le palmitate de rétinol, l'acétate de rétinol et le propionate de rétinol ainsi que leurs sels.

A titre d'exemple, les hydroxyacides, les céto-acides et les rétinoïdes peuvent être utilisés dans les compositions selon l'invention en une quantité représentant de 0,1 à 5 % en poids du poids total de la composition et mieux de 0,5 à 3 %.

Un test *in vitro* d'efficacité de la stimulation du renouvellement cellulaire épidermique par les composés selon l'invention a été réalisé sur *stratum corneum* en utilisant de l'acide salicylique comme composé de référence. Le principe du test repose sur le fait que la stimulation du renouvellement cellulaire épidermique induit la libération de cornéocytes. Le pouvoir de stimulation du renouvellement cellulaire épidermique du produit testé va être d'autant plus grand que le nombre de cornéocytes libérés et donc comptés sera important.

Le protocole du test a été le suivant : Chaque produit à la concentration de 3,8 millimoles dans un solvant formé de PEG-400, éthanol et eau (50/30/20) est testé sur trois échantillons de *stratum corneum* reconstitué, chaque échantillon consistant en une pastille de 8 mm de diamètre. Le produit est laissé en contact avec le *stratum corneum* pendant 4 heures. Avant le comptage des cornéocytes détachés, on agite fortement le tube contenant le *stratum corneum,* puis on effectue le comptage des cornéocytes détachés en cellules de Mallasez aux temps 4 heures et 24 heures. On calcule le nombre moyen des cornéocytes détachés à partir des trois résultats obtenus pour chaque produit.

La même mesure est réalisée pour un témoin sans actif ne contenant que du solvant car l'expérience produit inévitablement la libération de cornéocytes, même en absence d'actif.

Les résultats sont rassemblés dans le tableau suivant :

| Actif | Nombre moyen de cornéocytes à T 4 heures | Nombre moyen de cornéocytes à T 24 heures |
|---|---|---|
| Solvant | 2,67 | 5,00 |
| Acide salicylique | 13,33 | 9,33 |
| Acide gentisique | 8,00 | 13,00 |
| Acide dihydroxy-2,3-benzoïque | 14,64 | 18,33 |

Ces résultats montrent que l'acide gentisique et surtout l'acide dihydroxy-2,3-benzoïque ont à long terme de meilleures propriétés de stimulation du processus de renouvellement cellulaire épidermique que l'acide salicylique, en particulier à T 24 heures.

L'invention a encore pour objet un procédé de traitement cosmétique et/ou dermatologique des rides et/ou ridules et/ou taches de la peau consistant à appliquer sur les rides et/ou ridules et/ou taches une composition contenant une quantité efficace d'au moins un acide dihydroxybenzoïque.

La composition de l'invention contient un milieu physiologiquement acceptable, et donc cosmétiquement ou dermatologiquement acceptable, c'est-à-dire un milieu compatible avec la peau, les ongles, les muqueuses, les tissus et les cheveux. La composition contenant l'acide dihydroxybenzoïque peut être appliquée par voie topique sur le visage, le cou, les cheveux, les muqueuses et les ongles ou toute autre zone cutanée du corps.

Les compositions selon l'invention peuvent se présenter sous toutes les formes appropriées pour une application topique, notamment sous forme de solutions aqueuses, hydroalcooliques ou huileuses, de dispersions du type lotion ou sérum, de gels aqueux, anhydres ou huileux, d'émulsions de consistance liquide ou semi-liquide du type lait, obtenues par dispersion d'une phase grasse dans une phase aqueuse (H/E) ou inversement (E/H), de suspensions ou d'émulsions de consistance molle, semi-solide ou solide du type crème, gel, de microémulsions, ou encore de microcapsules, de microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage de la peau.

Les compositions peuvent également consister en des préparations solides constituant des savons ou des pains de nettoyage.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et éventuellement le coémulsionnant sont présents, dans la composition, en une proportion allant de 0,3 à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la quantité d'huile peut aller jusqu'à plus de 90 % en poids du poids total de la composition.

De façon connue, la composition de l'invention peut contenir également des adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans les domaines considérés, et par exemple de 0,01 à 15 % du poids total de la composition. Ces adjuvants, selon leur nature, peuvent être introduits dans la phase grasse, dans la phase aqueuse et/ou dans les vésicules lipidiques.

Comme huiles utilisables dans l'invention, on peut citer les huiles minérales (huile de vaseline), les huiles végétales (huile d'avocat, huile de soja), les huiles animales, les huiles de synthèse, les huiles siliconées (cyclométhicone, diméthicone) et les huiles fluorées (perfluoropolyéthers). On peut aussi utiliser comme matières grasses des alcools gras (alcool cétylique), des acides gras, des cires.

Comme émulsionnants utilisables dans l'invention, on peut citer par exemple les esters d'acide gras et de polyol, tels que les esters gras de sorbitol, notamment les Polysorbates et par exemple le Polysorbate 60, ou encore les esters gras de glycérol tels que le monostéarate de glycérol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides (méthylcellulose), les gommes naturelles et les argiles, et, comme gélifiants lipophiles, on peut citer les argiles modifiées comme les bentones, les sels métalliques d'acides gras, la silice hydrophobe et les polyéthylènes.

Comme actifs hydrophiles, on peut utiliser les protéines ou les hydrolysats de protéine, les acides aminés, les polyols (glycérine, propylène glycol), l'urée, l'allantoïne, les sucres et les dérivés de sucre, les vitamines hydrosolubles, l'amidon, les extraits bactériens ou végétaux, notamment d'Aloe Vera, les agents hydratants (acide hyaluronique).

Comme actifs lipophiles, on peut utiliser le tocophérol (vitamine E) et ses dérivés, les acides gras essentiels, les céramides, les huiles essentielles.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits sur la peau, le cuir chevelu et/ou les muqueuses.

Les exemples suivants illustrent l'invention. Dans ces exemples, les proportions indiquées sont des pourcentages en poids.

### Exemple 1 : Emulsion huile-dans eau

| *Phase huileuse :* | |
|---|---|
| Polysorbate 60 | 2 % |
| Monostéarate de glycérol | 2 % |
| Alcool cétylique | 1 % |
| Cyclométhicone | 2 % |
| Diméthicone | 1 % |
| Huile d'avocat | 2 % |
| Huile de soja | 3 % |

| *Phase aqueuse :* | |
|---|---|
| Acide gentisique | 0,5 % |
| Polyacrylamide | 1 % |
| Glycérol | 3 % |
| Méthylcellulose | 0,5 % |
| Conservateurs | 0,3 % |
| Eau déminéralisée | qsp 100 % |

Pour préparer l'émulsion, on mélange à chaud (environ 80°C) les composés de la phase huileuse d'une part et les composés de la phase aqueuse d'autre part, et on introduit la phase huileuse dans la phase aqueuse sous agitation pendant 15 minutes, puis on refroidit jusqu'à température ambiante sous agitation lente. On obtient une crème de jour qui provoque la stimulation du processus de renouvellement épidermique et la régénération de la peau et confère ainsi à celle-ci un aspect plus lisse et plus jeune qu'avant le traitement.

### Exemple 2 : Gel

| | |
|---|---|
| Acide gentisique | 1 % |
| Polyacrylamide | 1,5 % |
| Glycérol | 5 % |
| Conservateur | 0,3 % |
| Acide hyaluronique | 0,1 % |
| Propylène glycol | 2 % |
| Eau de rose | 10 % |
| Eau déminéralisée | qsp 100 % |

On obtient un gel qui, en application régulière deux fois par semaine, permet de lisser la peau tout en l'hydratant sans l'irriter.

### Exemple 3 : Lotion

| | |
|---|---|
| Acide gentisique | 0,3 % |
| Acide glycolique | 0,3 % |
| Glycérol | 1 % |
| Propylène glycol | 3 % |
| Eau d'hamamélis | 5 % |
| Alcool éthylique | 5 % |
| Conservateur | 0,3 % |
| Eau déminéralisée | qsp 100 % |

On obtient une lotion apte à traiter les peaux grasses par stimulation du processus de renouvellement épidermique et hydratation.

## Revendications

1. Utilisation cosmétique d'au moins un acide dihydroxybenzoïque dans une composition cosmétique pour stimuler le processus de renouvellement épidermique et/ou pour lutter contre les rides et/ou les ridules et/ou les taches actiniques et/ou les dyschromies cutanées et/ou l'acné.

2. Utilisation d'au moins un acide dihydroxybenzoïque pour la fabrication d'une composition dermatologique pour stimuler le processus de renouvellement épidermique et/ou pour lutter contre les rides et/ou les ridules et/ou les taches actiniques et/ou les dyschromies cutanées et/ou l'acné.

3. Utilisation selon la revendication 1 ou 2, caractérisée en ce que l'acide dihydroxybenzoïque est un composé de formule : 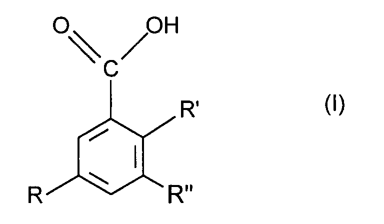 dans laquelle R, R' et R'' représentent indépendamment l'un de l'autre un hydrogène, un hydroxyle, un groupe alkyle ou un groupe alcoxy, deux au moins des radicaux R, R' et R'' représentant un hydroxyle.

4. Utilisation selon l'une quelconque des revendications précédentes, caractérisée en ce que l'acide dihydroxybenzoïque est choisi dans le groupe comprenant l'acide gentisique et l'acide dihydroxy-2,3-benzoïque.

5. Utilisation selon l'une quelconque des revendications précédentes, caractérisée en ce que l'acide dihydroxybenzoïque est utilisé en une quantité allant de 0,1 à 30 % en poids par rapport au poids total de la composition.

6. Utilisation selon l'une quelconque des revendications précédentes, caractérisée en ce que la composition comprend, en outre, au moins un actif choisi parmi les α-hydroxy-acides, les β-hydroxy-acides, les a-céto-acides, les β-céto-acides, les rétinoïdes.

7. Utilisation selon l'une quelconque des revendications précédentes, caractérisée en ce que la composition comprend, en outre, au moins un actif choisi parmi les acides glycolique, lactique, malique, tartrique, citrique, hydroxy-2 alcanoïque, mandélique, salicylique, l'acide n-octanoyl-5-salicylique.

8. Utilisation selon la revendication 6 ou 7, caractérisée en ce que l'actif est présent en une quantité allant de 0,1 à 5 % en poids par rapport au poids total de la composition.

9. Utilisation selon l'une quelconque des revendications précédentes, caractérisée en ce que la composition contient, en outre, au moins un adjuvant choisi parmi les protéines ou les hydrolysats de protéine, les acides aminés, les polyols, l'urée, les sucres et les dérivés de sucre, les vitamines, l'amidon, les extraits végétaux, les acides gras essentiels, les céramides, les huiles essentielles.

10. Utilisation selon l'une quelconque des revendications précédentes, caractérisée en ce que la composition est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules.

11. Procédé de traitement cosmétique des rides et/ou ridules et/ou taches de la peau consistant à appliquer sur les rides et/ou ridules et/ou taches, une composition contenant une quantité efficace d'au moins un acide dihydroxybenzoïque.

12. Composition cosmétique et/ou dermatologique, caractérisée en ce qu'elle contient, dans un milieu physiologiquement acceptable, au moins un acide dihydroxybenzoïque et au moins un actif choisi parmi les α-hydroxyacides, les β-hydroxy-acides, les α-céto-acides, les β-céto-acides, les rétinoïdes.

13. Composition selon la revendication 12, caractérisée en ce que l'acide dihydroxybenzoïque est un composé de formule : 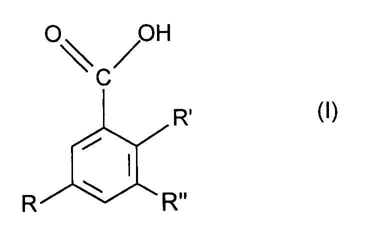 dans laquelle R, R' et R'' représentent indépendamment l'un de l'autre un hydrogène, un hydroxyle, un groupe alkyle ou un groupe alcoxy, deux au moins des radicaux R, R' et R'' représentant un hydroxyle.

14. Composition selon la revendication 12 ou 13, caractérisée en ce que l'actif est choisi parmi les acides glycolique, lactique, malique, tartrique, citrique, hydroxy-2 alcanoïque, mandélique, salicylique, l'acide n-octanoyl-5-salicylique.

## Claims

1. Cosmetic use of at least one dihydroxybenzoic acid in a cosmetic composition to stimulate the process of epidermal renewal and/or to combat wrinkles and/or fine lines, and/or actinic blemishes and/or skin dyschromias and/or acne.

2. Use of at least one dihydroxybenzoic acid for the manufacture of a dermatological composition to stimulate the process of epidermal renewal and/or to combat wrinkles and/or fine lines and/or actinic blemishes and/or skin dyschromias and/or acne.

3. Use according to Claim 1 or 2, characterized in that the dihydroxybenzoic acid is a compound of formula: 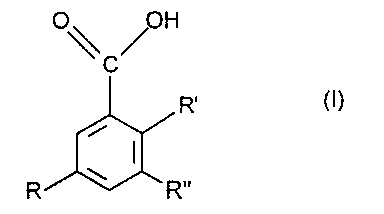 in which R, R' and R'' represent, independently of each other, a hydrogen, a hydroxyl, an alkyl group or an alkoxy group, at least two of the radicals R, R' and R'' representing a hydroxyl.

4. Use according to any one of the preceding claims, characterized in that the dihydroxybenzoic acid is chosen from the group comprising gentisic acid and 2,3-dihydroxybenzoic acid.

5. Use according to any one of the preceding claims, characterized in that the dihydroxybenzoic acid is used in an amount in the range from 0.1 to 30 % by weight relative to the total weight of the composition.

6. Use according to any one of the preceding claims, characterized in that the composition also comprises at least one active agent chosen from α-hydroxy acids, β-hydroxy acids, α-keto acids, β-keto acids and retinoids.

7. Use according to any one of the preceding claims, characterized in that the composition also comprises at least one active agent chosen from glycolic acid, lactic acid, malic acid, tartaric acid, citric acid, 2-hydroxyalkanoic acids, mandelic acid, salicylic acid and 5-n-octanoylsalicylic acid.

8. Use according to Claim 6 or 7, characterized in that the active agent is present in an amount in the range from 0.1 to 5 % by weight relative to the total weight of the composition.

9. Use according to any one of the preceding claims, characterized in that the composition also contains at least one adjuvant chosen from proteins or protein hydrolysates, amino acids, polyols, urea, sugars and sugar derivatives, vitamins, starch, plant extracts, essential fatty acids, ceramides and essential oils.

10. Use according to any one of the preceding claims, characterized in that the composition is an aqueous, oily or aqueous-alcoholic solution, a water-in-oil emulsion, an oil-in-water emulsion, a microemulsion, an aqueous gel, an anhydrous gel, a serum or a dispersion of vesicles, of microcapsules or of microparticles.

11. Process for the cosmetic treatment of wrinkles and/or fine lines and/or blemishes of the skin, which consists in applying a combination containing an effective amount of at least one dihydroxybenzoic acid to the wrinkles and/or fine lines and/or blemishes.

12. Cosmetic and/or dermatological composition, characterized in that it contains, in a physiologically acceptable medium, at least one dihydroxybenzoic acid and at least one active agent chosen from α-hydroxy acids, β-hydroxy acids, α-keto acids, β-keto acids and retinoids.

13. Composition according to Claim 12, characterized in that the dihydroxbenzoic acid is a compound of formula: 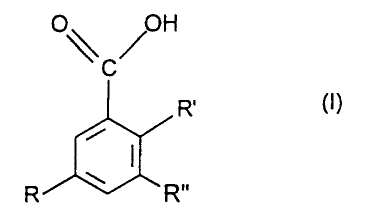 in which R, R' and R'' represent, independently of each other, a hydrogen, a hydroxyl, an alkyl group or an alkoxy group, at least two of the radicals R, R' and R'' representing a hydroxyl.

14. Composition according to Claim 12 or 13, characterized in that the active agent is chosen from glycolic acid, lactic acid, malic acid, tartaric acid, citric acid, 2-hydroxyalkanoic acids, mandelic acid, salicylic acid and 5-n-octanoylsalicylic acid.

## Patentansprüche

1. Kosmetische Verwendung mindestens einer Dihydroxybenzoesäure in einer kosmetischen Zusammensetzung zur Stimulierung des Prozesses der Epidermiserneuerung und/oder zur Bekämpfung von Falten und/oder Fältchen und/oder durch aktinische Strahlung hervorgerufenen Flecken und/oder Dyschromien der Haut und/oder Akne.

2. Verwendung mindestens einer Dihydroxybenzoesäure zur Herstellung einer dermatologischen Zusammensetzung zur Stimulierung des Prozesses der Epidermiserneuerung und/oder zur Bekämpfung von Falten und/oder Fältchen und/oder durch aktinische Strahlung hervorgerufenen Flecken und/oder Dyschromien der Haut und/oder Akne.

3. Verwendung nach Anspruch 1 oder 2, dadurch gekennzeichnet, daß die Dihydroxybenzoesäure eine Verbindung der folgenden Formel ist: 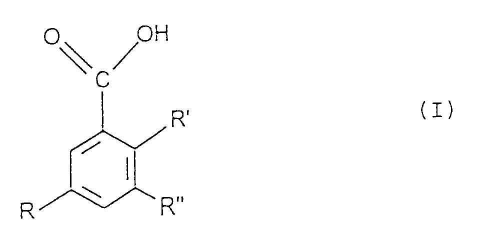 worin R, R' und R'' unabhängig voneinander Wasserstoff, Hydroxy, Alkyl oder Alkoxy bedeuten, wobei mindestens zwei der Gruppen R, R' und R'' Hydroxy bedeuten.

4. Verwendung nach einem der vorhergehenden Ansprüche, dadurch gekennzeichnet, daß die Dihydroxybenzoesäure unter Gentisinsäure und 2,3-Dihydroxybenzoesäure ausgewählt ist.

5. Verwendung nach einem der vorhergehenden Ansprüche, dadurch gekennzeichnet, daß die Dihydroxybenzoesäure in einem Mengenanteil von 0,1 bis 30 Gew.-%, bezogen auf das Gesamtgewicht der Zusammensetzung, verwendet wird.

6. Verwendung nach einem der vorhergehenden Ansprüche, dadurch gekennzeichnet, daß die Zusammensetzung ferner mindestens einen Wirkstoff enthält, der unter den α-Hydroxysäuren, β-Hydroxysäuren, α-Ketosäuren, β-Ketosäuren und Retinoiden ausgewählt ist.

7. Verwendung nach einem der vorhergehenden Ansprüche, dadurch gekennzeichnet, daß die Zusammensetzung ferner mindestens einen Wirkstoff enthält, der unter Glykolsäure, Milchsäure, Äpfelsäure, Weinsäure, Citronensäure, 2-Hydroxyalkancaronsäuren, Mandelsäure, Salicylsäure und 5-(n-Octanoyl)-salicylsäure ausgewählt ist.

8. Verwendung nach Anspruch 6 oder 7, dadurch gekennzeichnet, daß der Wirkstoff in einem Mengenanteil von 0,1 bis 5 Gew.-%, bezogen auf das Gesamtgewicht der Zusammensetzung, vorliegt.

9. Verwendung nach einem der vorhergehenden Ansprüche, dadurch gekennzeichnet, daß die Zusammensetzung ferner mindestens einen Zusatzstoff enthält, der unter den Proteinen oder Proteinhydrolysaten, Aminosäuren, Polyolen, Harnstoff, Zuckern und Zuckerderivaten, Vitaminen, Stärke, Pflanzenextrakten, essentiellen Fettsäuren, Ceramiden und etherischen Ölen ausgewählt ist.

10. Verwendung nach einem der vorhergehenden Ansprüche, dadurch gekennzeichnet, daß die Zusammensetzungen wäßrige, ölige oder wäßrig-alkoholische Lösungen, Wasser-in-Öl-Emulsionen, Öl-in-Wasser-Emulsionen, Mikroemulsionen, wäßrige Gele, wasserfreie Gele, Seren, Vesikeldispersionen, Mikrokapseln oder Mikropartikel sind.

11. Verfahren zur kosmetischen Behandlung von Falten und/oder Fältchen und/oder Flecken der Haut, das darin besteht, auf die Falten und/oder Fältchen und/oder Flecken eine Zusammensetzung aufzutragen, die eine wirksame Menge mindestens einer Dihydroxybenzoesäure enthält.

12. Kosmetische und/oder dermatologische Zusammensetzung, die dadurch gekennzeichnet, daß sie in einem physiologisch akzeptablen Medium mindestens eine Dihydroxybenzoesäure und mindestens einen Wirkstoff enthält, der unter den α-Hydroxysäuren, β-Hydroxysäuren, α-Ketosäuren, β-Ketosäuren und Retinoiden ausgewählt ist.

13. Zusammensetzung nach Anspruch 12, dadurch gekennzeichnet, daß die Dihydroxybenzoesäure eine Verbindung der folgenden Formel ist: 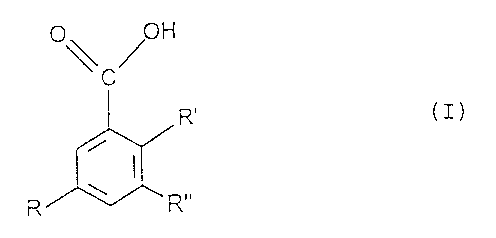 worin R, R' und R'' unabhängig voneinander Wasserstoff, Hydroxy, Alkyl oder Alkoxy bedeuten, wobei mindestens zwei der Gruppen R, R' und R'' Hydroxy bedeuten.

14. Zusammensetzung nach Anspruch 12 oder 13, dadurch gekennzeichnet, daß der Wirkstoff unter Glykolsäure, Milchsäure, Äpfelsäure, Weinsäure, Citronensäure, 2-Hydroxyalkancarbonsäuren, Mandelsäure, Salicylsäure und 5-(n-Octanoyl)-Salicylsäure ausgewählt ist.
